# EUROPEAN PATENT APPLICATION

(11) **EP 4 464 795 A1**
(43) Date of publication of application: **20.11.2024**
(21) Application number: 22920518.2
(22) Date of filing: 08.12.2022
(51) Int. Cl.: C12Q 1/6853, C12N 15/09, C12N 15/11, C12Q 1/686, C12Q 1/6876

(54) **METHOD FOR DETECTING STRUCTURAL POLYMORPHISM MUTATIONS**

(30) Priority: 14.01.2022 JP 2022004599; 24.06.2022 JP 2022101921
(71) Applicant: Hitachi, Ltd., Tokyo 100-8280 (JP)
(72) Inventor: MANRI Chihiro, Tokyo 100-8280 (JP); YOKOI Takahide, Tokyo 100-8280 (JP); ANDO Takahiro, Tokyo 100-8280 (JP)
(74) Representative: Mewburn Ellis LLP
(86) International application number: PCT/JP2022/045353
(87) International publication number: WO 2023/135998

(57) **Abstract**

Provided is a method and a means for improving multiplex simultaneous detection of a structural polymorphism mutation in capillary electrophoresis. In one embodiment, the present invention relates to a method for detecting a mutation in a test nucleic acid, the method including: providing a first primer set, containing at least one labeled first primer, for amplifying a nucleic acid with a first mutation; providing a second primer set, containing at least one labeled second primer, for amplifying a nucleic acid with a second mutation; amplifying the test nucleic acid with use of the first primer set and the second primer set; and subjecting an obtained amplification product to capillary electrophoresis to analyze the mutation in the test nucleic acid, the first primer and the second primer being individually labeled with combinations of different numbers and/or kinds of fluorescent dyes.

## Description

### Technical Field

The present invention relates to a method and a means for detecting gene mutations. More specifically, the present invention relates to a method and a means for detecting a structural polymorphism mutation of gene, by capillary electrophoresis.

### Background Art

There are various individual differences in genome, and the difference in genome sequence (genomic mutation) can provide a biomarker useful as an index of disease or drug response. Major methods having been utilized for detection of the genomic mutation include detection based on polymerase chain reaction (PCR), sequencing analysis with use of a sequencer, and fragment analysis based on electrophoresis.

Recent progress in genomics has enabled a panel test of genomic mutation with use of a massively parallel sequencer capable of large-scale analysis. Parallel analyses of a large number of genetic mutations enable decision of a variety of diseases and therapies at a time. In particular, cancer screening by liquid biopsy is expected to greatly advance in the future. The liquid biopsy, which is a blood-based test, less invasive, can systemically screen cancer, and has therefore been investigated with an expectation as a new cancer screening test (NPL 1). Meanwhile, from the viewpoint of social implementation of the cancer screening test, the liquid biopsy has an issue of analysis cost. For the social implementation of the liquid biopsy, it is necessary to develop a low-cost, multi-mutation test technology, which can substitute the expensive massively parallel sequencer.

### Citation Lists

### Patent Literature

PTL 1: US 2014/0213471 A

### Non-Patent Literatures

NPL 1: Cohen, J.D. et al., Science, Vol. 359, p.926-930 (2018)
NPL 2: Pereira, R. et al., PLOS ONE, Vol. 7, No. 1, e29684 (2012)
NPL 3: Ren, X. et al., Nucleic Acids Research, Vol. 44, No. 8, e79 (2016)

### Summary of Invention

### Technical Problem

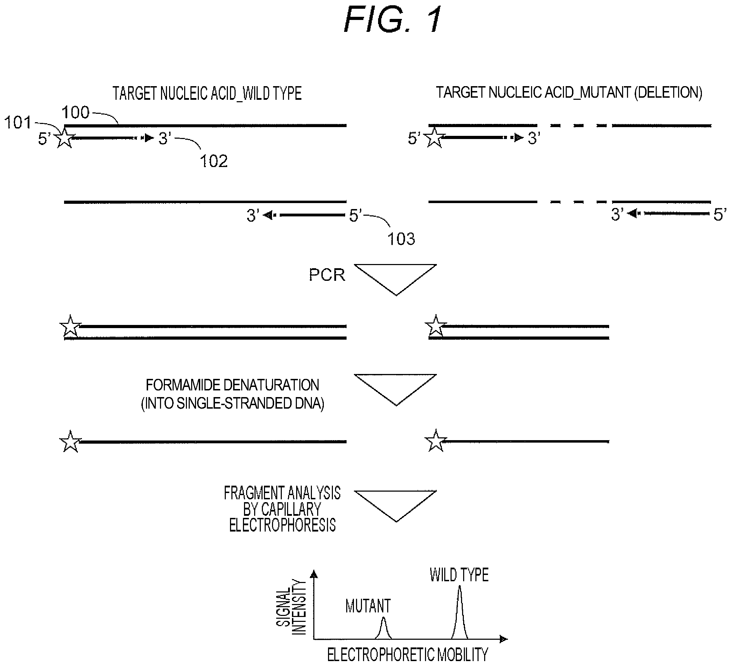

Fragment analysis based on capillary electrophoresis (CE) has been known as an exemplary technology capable of detecting a wide variety of genetic mutations at low cost (FIG. 1). The method uses a forward primer 102 and a reverse primer 103, both designed complementary to the sequence of a target nucleic acid 100 that contains a mutation to be detected. Either primer has a fluorescent dye 101, which is distinguishable for every target nucleic acid, previously labeled at the 5' end of the primer. PCR when conducted with use of such primer set can amplify not only a normal target nucleic acid (wild type), but also a nucleic acid with mutation (structural mutation such as deletion, insertion, or fusion, in which FIG. 1 illustrates deletion) (mutant), if exists, at the same time. After amplified and dissociated into a single-stranded DNA, the DNA is subjected to electrophoretic separation by capillary electrophoresis, in which the mutant target nucleic acid is finally detected by fluorescence of the fluorescent dye bound to the 5' end. The deletion mutation, which yields a shorter amplification product, migrates faster as compared with the wild-type nucleic acid. The insertion mutation, which yields a longer amplification product, migrates slower as compared with the wild-type nucleic acid. The fusion mutation, which can yield both of longer and shorter amplification products, demonstrates a mobility different from the wild type. As described above, the mutant, if contained, demonstrates phoretic mobility different from that of the wild type, thereby enabling detection of the mutant.

This method, whose detection relies upon the fluorescent dye labeled at the 5' end, enables multiplex simultaneous detection by changing the fluorescent dye to be used. For example, a literature by Pereira et al. (NPL 2) describes simultaneous detection of structural polymorphism with use of four types of fluorescent dye. The number of genes that can be detected at a time is, however, limited to the number of types of fluorescent dye, which is only several genes as much.

In addition, mutant gene contained in human body fluid used for liquid biopsy, whose frequency of appearance has been known to be as low as several percent (approximately 0.1 to 1.0%) as compared with the wild type, is not easily detectable by capillary electrophoresis.

Moreover, the structural polymorphism mutation, having various base lengths (sizes), would be difficult to determine whether a signal detected in the capillary electrophoresis is derived from the target nucleic acid or not.

Furthermore, the fusion mutation will not suffice if it is simply detected, since the therapy varies depending on a paired gene to be fused (and also required identification of the paired gene).

That is, how a less abundant mutant is detectable without being limited by the number of existing fluorescent dyes, is necessary to extend ability of the multiplex simultaneous detection of the structural polymorphism mutations. Furthermore, it is also important to certify that the acquired signal is derived from the target gene. It is therefore an object of the present invention to provide a method and a means for improving multiplex simultaneous detection of structural polymorphism mutation(s) in capillary electrophoresis.

Note that a method has been reported, according to which a probe is multiply labeled with a plurality of fluorescent dyes to detect mutant genes in a cell or a tissue (NPL 3). There is, however, no description on the primer. Another method has been reported, according to which a polynucleotide to be analyzed is detected with use of a primer labeled with a fluorescent dye (PTL 1). There is, however, no description on the multi-labeling.

### Solution to Problem

The present inventors have found that, in genetic analysis with use of capillary electrophoresis (CE), use of primers individually labeled with different numbers and/or combinations of fluorescent dye enables simultaneous analysis and detection of different types of mutations (particularly structural polymorphism mutation). The present inventors also found that combination of the plurality of fluorescent dyes can enhance the fluorescence intensity, and can enable discriminable detection of less-frequent mutation by CE. The present invention has been made in consideration of these findings.

In one embodiment, the present invention provides a method for detecting a mutation in a test nucleic acid, the method including:
providing a first primer set, containing at least one labeled first primer, for amplifying a nucleic acid with a first mutation;
providing a second primer set, containing at least one labeled second primer, for amplifying a nucleic acid with a second mutation;
amplifying the test nucleic acid with use of the first primer set and the second primer set; and
subjecting an obtained amplification product to capillary electrophoresis to analyze the mutation in the test nucleic acid,
the first primer and the second primer being individually labeled with combinations of different numbers and/or kinds of fluorescent dyes.

In another embodiment, the present invention provides a kit used for detecting a mutation in a nucleic acid by capillary electrophoresis, the kit including:
a first primer set, containing at least one labeled first primer, for amplifying a nucleic acid with a first mutation; and
a second primer set, containing at least one labeled second primer, for amplifying a nucleic acid with a second mutation,
the first primer and the second primer being individually labeled with combinations of different numbers and/or kinds of fluorescent dyes.

### Advantageous Effects of Invention

The present invention can provide a method and means capable of simultaneously detecting a plurality of structural polymorphism mutations. The present invention also enables discriminable detection of less frequent mutation by CE. The present invention can, therefore, detect more structural polymorphism mutations at a time and with high sensitivity.

### Brief Description of Drawings

[FIG. 1] FIG. 1 is a schematic drawing illustrating a fragment analysis method based on capillary electrophoresis.
[FIG. 2] FIG. 2 is a schematic drawing illustrating a fragment analysis method based on capillary electrophoresis, according to Example 1.
[FIG. 3] FIG. 3 shows (A)table listing primers labeled with one or more fluorescent dyes, and (B) a fluorescence spectral chart obtained with use of these primers.
[FIG. 4] FIG. 4 is a graph showing that multi-labeling of primers individually with a plurality of fluorescent dyes can enhance fluorescence intensity.
[FIG. 5] FIG. 5 shows (A) table listing primers individually labeled with a different combination of fluorescent dyes for different target nucleic acids, and (B) a fluorescence spectral chart of amplified products obtained with use of these primers, measured after phoresis.
[FIG. 6] FIG. 6 is a schematic drawing illustrating a fragment analysis method based on capillary electrophoresis, according to Example 2.
[FIG. 7] FIG. 7 is a schematic drawing illustrating a conventional fragment analysis method based on capillary electrophoresis.
[FIG. 8] FIG. 8 is a schematic drawing illustrating a fragment analysis method based on capillary electrophoresis, according to Example 3.
[FIG. 9A] FIG. 9A is a table listing primer sets and labels for detecting ROS1-SLC34A2 gene which is a target sequence having gene fusion mutation.
[FIG. 9B] FIG. 9B shows graphs (fluorescence spectral charts of amplified products after phoresis) showing effects of detection of fusion mutations by multi-labeling with use of a forward primer and a reverse primer both labeled with fluorescence.
[FIG. 10] FIG. 10 is a schematic drawing illustrating a fragment analysis method based on capillary electrophoresis, according to Example 4.
[FIG. 11A] FIG. 11A is a graph showing effects of detection of fusion mutations by multi-labeling with use of a forward primer and a reverse primer both labeled with fluorescence.
[FIG. 11B] FIG. 11B shows graphs (fluorescence spectral charts of amplified products after phoresis under non-denaturating conditions) showing effects of detection of fusion mutations by multi-labeling with use of a forward primer and a reverse primer both labeled with fluorescence.

### Description of Embodiments

This application claims priority to Japanese Patent Application No. 2022-004599 filed on January 14, 2022, and Japanese Patent Application No. 2022-101921 filed on June 24, 2022, the contents of which are incorporated herein by reference in their entirety.

The present invention is based on use of primers labeled with a combination of different numbers and/or types of fluorescent dyes to detect different gene mutations, upon detection of gene mutations by fragment analysis technique with use of capillary electrophoresis.

In one embodiment, the present invention relates to a method for detecting a mutation in a test nucleic acid, the method including:
providing a first primer set, containing at least one labeled first primer, for amplifying a nucleic acid with a first mutation;
providing a second primer set, containing at least one labeled second primer, for amplifying a nucleic acid with a second mutation;
amplifying the test nucleic acid with use of the first primer set and the second primer set; and
subjecting an obtained amplification product to capillary electrophoresis to analyze the mutation in the test nucleic acid,
the first primer and the second primer being individually labeled with combinations of different numbers and/or kinds of fluorescent dyes.

In the present invention, detecting a mutation in a test nucleic acid means to determine whether or not a mutation to be detected is present in the test nucleic acid, and if present, to determine natures of the mutation (wild type, mutant, etc.) and abundance ratio.

The mutation to be detected according to the present invention may not be particularly limited as long as it is a mutation of a gene. The present invention may particularly be suitable for detecting a structural polymorphism mutation (mutation causing change in the base length) of a gene. The structural polymorphism mutations typically include deletion (the base length shortened due to deletion of base), insertion (the base length elongated due to insertion of base), and fusion (base length varies depending on a mode of fusion with other gene). Examples of the mutation may include, but are not limited to, mutations of EGFR gene (mutation resulted from deletion of bases at positions from 746 to 750 in exon 19, mutation resulted from insertion of bases between positions 769 and 770 in exon 20), and mutations of ROS1 gene (mutations resulted from fusion of ROS1 with another gene (SLC34A2, CD74, SDC4, etc.)). Although the identity of the mutation may be referred herein to as wild type or mutant for convenience, the mutation is not limited by the terms wild type and mutant, since some mutation does not have wild type, or has a plurality of mutants, thus causing two or more polymorphs in a single mutation.

According to the present invention, a primer set for amplifying a nucleic acid containing a mutation to be detected may be provided, in which each primer set contains at least one labeled primer. In one embodiment, aimed at detecting two different mutations, a first primer set, containing a labeled first primer, for amplifying a nucleic acid with a first mutation; and a second primer set, containing a labeled second primer, for amplifying a nucleic acid with a second mutation may be used.

In another embodiment, aimed at detecting three different mutations, a first primer set, containing a labeled first primer, for amplifying a nucleic acid with a first mutation; a second primer set, containing a labeled second primer, for amplifying a nucleic acid with a second mutation; and a third primer set, containing a labeled third primer, for amplifying a nucleic acid with a third mutation may be used.

Similarly, aimed at detecting n different mutations, n sets of an n-th primer sets for amplifying a nucleic acid with an n-th mutation, each containing at least a labeled n-th primer may be included. Where, n represents an integer of 3 to 100, whose maximum value is variable depending on detection ability of an instrument used for capillary electrophoresis (CE). Hence in one embodiment, the method of the present invention may further include providing an n-th primer set, containing at least one labeled n-th primer, for amplifying a nucleic acid with a mutation different from the first mutation and the second mutation, where n represents an integer of 3 to 100, and the n-th primer is labeled with a combination of fluorescent dyes whose number and/or kind are different from those in the first primer and the second primer.

According to the present invention, the primer (also referred herein to as forward primer or reverse primer) may be either DNA or RNA, which may be determined according to the type of the test nucleic acid, and the type of polymerase used for PCR. The primer may preferably be DNA, with which PCR is conducted with use of DNA or mRNA as the test nucleic acid which serves as a template.

The primer may be designed so as to enable specific binding and subsequent amplification of the target nucleic acid that contains a mutation, that is, to have a sequence complementary to the target nucleic acid. Design techniques of the primer are well known in the art, and the primers which can be used in the present invention may satisfy conditions allowed for specific annealing. For example, the primer may be designed to have a length and base composition (melting temperature) allowed for specific annealing. For example, base length of the primer which can function as a primer may be preferably 10 bases or larger, and more preferably 15 to 50 bases. When designed, the primer may preferably be checked in terms of the GC content and the melting temperature (Tm). Tm means a temperature at which 50% of any nucleic acid strand hybridizes with its complementary strand. The annealing temperature may necessarily be optimized, in order to anneal the target nucleic acid which serves as a template with the primer to form a double strand. The temperature may preferably be set high as possible, since the temperature if excessively lowered will induce non-specific reaction. Tm may be checked with use of any of known software for primer design. The designed primer may be chemically synthesized with use of any of known methods for oligonucleotide synthesis, and may usually be synthesized with use of a commercially available chemical synthesis apparatus.

The at least one primer contained in one primer set may be labeled with one or more fluorescent dyes. Examples of such fluorescent dyes may include, but not limited to, fluorescein (FITC), Cy dyes (e.g., Cy6), rhodamine 6G (R6G), sulforhodamine (TR), tetramethylrhodamine (TRITC), carboxy-X-rhodamine (ROX), carboxytetramethylrhodamine (TAMRA), NED, 5-carboxyfluorescein (5-FAM), 6-carboxyfluorescein (6-FAM), 5'-hexachlorofluorescein CE-phosphoramidite (HEX), 6-carboxy-4',5'-dichloro-2',7'-dimethoxyfluorescein (JOE), 5-tetrachlorofluorescein CE-phosphoramidite (TET), rhodamine 110 (R110), VIC (registered trademark), ATTO dyes, and Alexa Fluor (registered trademark). Examples of the fluorescent dye that do not cause shift (change) of phoretic size may include dR110 (carboxy-dichlororhodamine 110), dR6G (dihydro rhodamine 6G), dTAMRA (tetramethyl rhodamine), and dROX (carboxy-X-rhodamine).

According to the present invention, primers for detecting different mutations may be labeled with a combination of different numbers and/or types of fluorescent dyes. The combination of fluorescent dyes may be selected so that the amplified products bound with the fluorescent dye will demonstrate different mobilities in capillary electrophoresis. In an exemplary case where a plurality of different target nucleic acids (mutations) are detected, a plurality of fluorescent dyes excited and detected at different wavelengths may be combined and used.

When desired to intensify fluorescence signal, a primer may be used after labeled with two or more molecules of fluorescent dye. It may be preferred to use two or more molecules of the same kind of fluorescent dye.

The fluorescent dye may label the 5' end of the primer, and/or the base within (internal) the sequence of the primer. For example, the primer may be labeled with a fluorescent dye at the 5' end. Alternatively, the primer may be labeled with a fluorescent dye at the 5' end, and at one base within (Internal) the sequence of the primer. Alternatively, the primer may be labeled with a fluorescent dye at the 5' end, and at two or more bases within (Internal) the sequence of the primer. Alternatively, the primer may be labeled with a fluorescent dye at one or more bases within (internal of) the sequence of the primer.

Furthermore, both the forward primer and the reverse primer used herein may be labeled, in order to certify that the detected fluorescence signal is derived from the target nucleic acid (mutation). The fluorescent dyes used in this case may be of the same type or different types. Alternatively, one or both of the forward primer and the reverse primer may be labeled with a plurality of fluorescent dyes. Thus, in one embodiment, the first primer set may further include a labeled first' primer (besides the labeled first primer), and/or the second primer set may further include a labeled second' primer (besides the labeled second primer). In one embodiment, the first primer and the first' primer may also be individually labeled with a combination of the same number and/or kind of fluorescent dyes, or with combinations of different numbers and/or kinds of fluorescent dyes. In one embodiment, the second primer and the second' primer may also be individually labeled with a combination of the same number and/or kind of fluorescent dyes, or with combinations of different numbers and/or kinds of fluorescent dyes.

The labeling method may not be particularly limited, and conventional various known means may be applicable.

The test nucleic acid may not be particularly limited as long as it is a sample containing a nucleic acid, and any sample such as biological sample (for example, cell sample, tissue sample, fluid sample, etc.), and synthetic sample (for example, nucleic acid library such as cDNA library) may be applicable. The biological sample may not be particularly limited regarding the biological origin from which the sample is derived, and those derived from any biological bodies including vertebrate (for example, mammals, birds, reptiles, fish, amphibians, etc.), invertebrate (for example, insects, nematodes, crustaceans, etc.), protist, plant, fungus, bacterium, and virus may be used. For an exemplary case intended for cancer test in human, provided is nucleic acid-containing sample obtainable from a human to be tested, such as whole blood, serum, plasma, saliva, urine, feces, skin tissue, cancer tissue, or the like. For example, the test nucleic acid may be a nucleic acid sample containing, or suspected of containing a target nucleic acid (mutation).

The test nucleic acid may not be particularly limited as long as it is a nucleic acid containing a mutation to be detected, and may include deoxyribonucleic acid (DNA) such as genomic DNA and cDNA, and ribonucleic acid (RNA) such as messenger RNA (mRNA) and fragments thereof. The test nucleic acid preferably used in the present invention may be, for example, cell-free DNA (cfDNA, which is free DNA in blood), or circulating tumor DNA (ctDNA). The nucleic acids may be prepared from the sample by any of methods known in the art. The test nucleic acid may be prepared from blood or cell, typically by lysing cell with use of proteolytic enzyme such as proteinase K; chaotropic salt such as guanidine thiocyanate and guanidine hydrochloride; surfactant such as Tween and SDS; or commercially available cell lysis reagent, so as to elute nucleic acid, that is, DNA and RNA contained therein. RNA may be prepared by lysing DNA, from among the nucleic acids eluted by the cell lysis, with use of a deoxyribonuclease (DNase), by which a sample solely containing RNA as the nucleic acid is obtainable. mRNA, which contains poly-A sequence, may be prepared from the thus prepared RNA sample, with use of a DNA probe that contains a poly-T sequence, thereby capturing mRNA only. There are kits for preparing nucleic acid marketed from many manufacturers, with which a desired nucleic acid may be purified easily.

Next, the test nucleic acid may be amplified with use of the first primer set and the second primer set. That is, an amplification reaction with use of the primer sets, preferably a polymerase chain reaction (PCR), may be conducted with use of the test nucleic acid as a template. PCR has been known in the art, in which a polymerase used therefor may be selected depending on types of the template (test nucleic acid) and the primers to be used. For example, for PCR with use of a DNA template or an RNA template together with a DNA primer, a DNA-dependent DNA polymerase or an RNA-dependent DNA polymerase may be used, respectively.

According to the presence of the test nucleic acid, the primer that specifically binds to the test nucleic acid may hybridize therewith, and the primer may be extended from the 3' end by incorporating a base(s) as a substrate(s), aided by a synthetic reaction by the polymerase. Repetition of such extension amplifies the test nucleic acid. Presence or absence of the test nucleic acid may be determined by whether or not the amplification reaction occurs, meanwhile the type of mutation in the test nucleic acid may be determined with reference to the type (combination) of the label on the primer. Presence or absence of mutation may be detected with reference to the phoretic mobility in the capillary electrophoresis.

After amplified by PCR, the obtained reaction product may be analyzed by capillary electrophoresis (CE). The obtained amplified product may alternatively be dissociated, after the amplification of the test nucleic acid, into a single-stranded nucleic acid, and then may be subjected to CE. Methods for dissociating the amplified product (double-stranded nucleic acid) to produce single strands are known in the art, which may typically rely upon alkali denaturation treatment or high temperature treatment.

The capillary electrophoresis (CE) is a technique of separating an injected component typically according to mobility difference due to electric charge, size or shape, and of detecting the components with the aid of the labeling substance. With use of the multi-labeled primers having different numbers and/or types of labeling dyes, the present method can detect a nucleic acid that contains a mutation of interest and/or can determine the presence or absence of a mutation in the nucleic acid, with reference to the presence or absence of a mutation of interest or the type of specific label (based on a multi-labeled primer labeled with one or more fluorescent dyes), and with reference to the type of mutation of the test nucleic acid (based on structural polymorphism of the nucleic acid) judged from the mobility.

The method of the present invention can detect a plurality of types (1 to 100 types, for example) of mutation at a time, both in terms of presence and type, with high sensitivity even if the mutation is less abundant.

The method of the present invention may be more easily and simply implemented, with use of a kit that includes a plurality of primers labeled with combinations of different numbers and/or types of fluorescent dyes. That is, in another aspect, the present invention relates to a kit used for detecting a mutation in a test nucleic acid by capillary electrophoresis, the kit including:
a first primer set, containing at least one labeled first primer, for amplifying a nucleic acid with a first mutation; and
a second primer set, containing at least one labeled second primer, for amplifying a nucleic acid with a second mutation,
the first primer and the second primer being individually labeled with combinations of different numbers and/or kinds of fluorescent dyes.

In one embodiment, the first primer set may further include a labeled first' primer; and/or the second primer set may further include a labeled second' primer. The first primer and the first' primer in this case may individually be labeled with a combination of the same number and/or kind of fluorescent dyes, or with combinations of different numbers and/or kinds of fluorescent dyes. In addition, or alternatively, the second primer and the second' primer may individually be labeled with a combination of the same number and/or kind of fluorescent dyes, or with combinations of different numbers and/or kinds of fluorescent dyes.

In one embodiment, the 5' end of the first primer and/or the second primer may be labeled with a fluorescent dye; and/or a base within the first primer and/or the second primer may be labeled with a fluorescent dye.

The kit of the present invention may further include an n-th primer set, containing at least one labeled n-th primer, for amplifying a nucleic acid with a mutation different from the first mutation and the second mutation, where n represents an integer of 3 to 100, and the n-th primer may be labeled with a combination of fluorescent dyes whose number and/or kind are different from those in the first primer and the second primer.

The kit of the present invention may contain, in addition to the primer or the primer set, a buffer constituting the reaction solution, a dNTP or a ddNTP mixture (which may be labeled), enzymes (polymerase, reverse transcriptase, etc.), a standard sample for calibration, and the like. The primers provided in a form of kit may enable more quick and simple detection of genetic mutation.

Any constituent denoted herein in singular form is construed to encompass plural form, unless the context clearly dictates otherwise.

The present invention is not to be construed as being limited to the description in Examples below. Those skilled in the art can easily understand that the specific configuration may be modified without departing from the spirit or gist of the present invention.

Position, size, shape, range, and the like of the respective components illustrated in the drawings and the like may be different from the actual position, size, shape, range, and the like, for the convenience of understanding of the invention. The present invention is, therefore, not always necessarily limited to the position, size, shape, range, and the like disclosed in the drawings and the like.

### [Example 1]

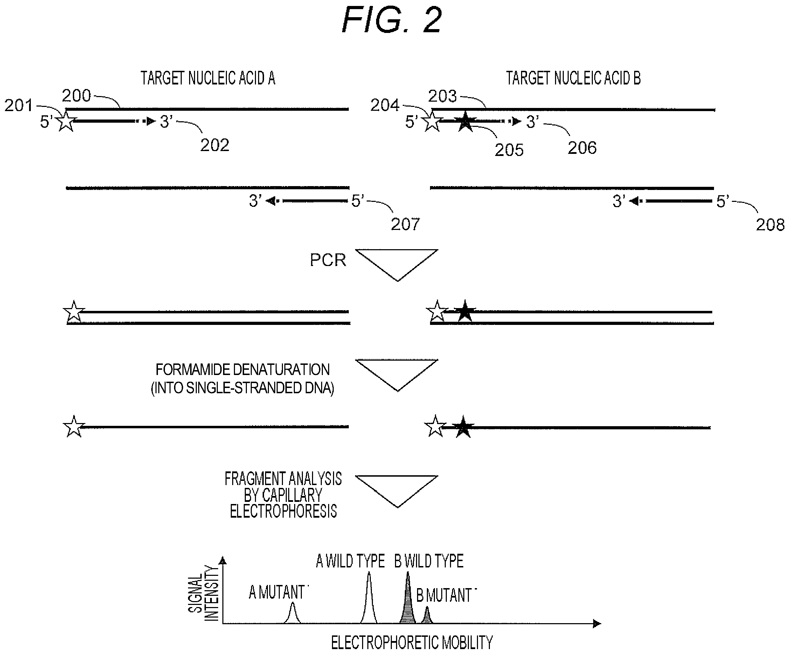

FIG. 2 is a schematic drawing illustrating a fragment analysis method based on capillary electrophoresis, according to Example 1. The drawing illustrates a case where two types of target nucleic acids A and B are contained in the same sample. Note that the number of types of the target nucleic acid is not limited to two.

Used herein are a forward primer 202 and a reverse primer 207 designed complementary to a target nucleic acid A 200 that contains a mutation to be detected; and a forward primer 206 and a reverse primer 208 designed complementary to a target nucleic acid B 203 that contains another mutation to be detected. The forward primer 202 has a fluorescent dye 201 labeled at the 5' end; meanwhile the forward primer 206 has a fluorescent dye 204 labeled at the 5' end, and another fluorescent dye 205 labeled within (Internal) the primer. The reverse primers are unlabeled. After amplified by PCR with use of such primers and dissociating into single-stranded DNAs, the DNAs are phoretically separated by the capillary electrophoresis (CE). A capillary for the capillary electrophoresis is packed with a polymer, and POP4 from Thermo Fisher Scientific K.K., for example, may be used. Fluorescence detection concurrently with the phoretic separation can finally detect different target nucleic acids and individually correspondent genetic mutations at a time. The drawing illustrates the target nucleic acid A whose mutation is deletion, and the target nucleic acid B whose mutation is insertion. Note that the reverse primers may alternatively be labeled, with the forward primers remained unlabeled. The fluorescent dyes 201, 204, and 205 may be of the same kind, or of different kinds. Combination of the fluorescent dyes may not be limited. The fluorescent dyes labeled at the 5' end and the internal site may be, however, preferably combined so as to demonstrate different fluorescence wavelengths when excited at the same wavelength.

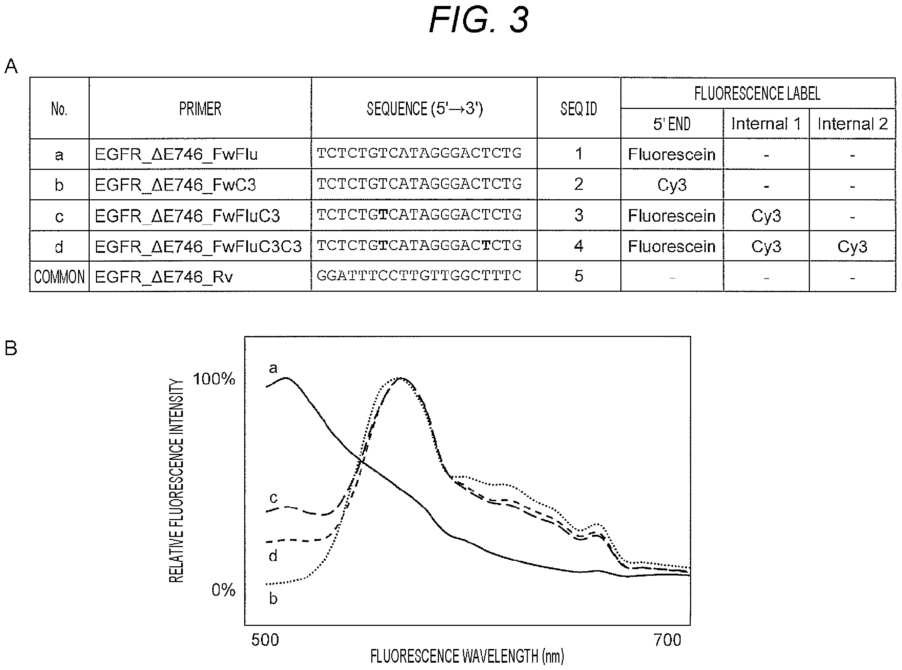

FIG. 3 shows effects of yielding different fluorescence spectra as a result of the multi-labeling of the primers. The target nucleic acid used herein contains EGFR gene, known to be a genetic mutation that is frequently found in colorectal cancer and lung cancer, as a target sequence, for which four forward primers capable of detecting a genetic mutation with deletion at positions from 746 to 750 were prepared (see FIG. 3A), which include primers having either fluorescein (a) or Cy3 (b) at the 5' end; and primers individually having fluorescein labeled at the 5' end, together with one and two molecules of Cy3 (c) and (d), respectively, labeled within thereof. The reverse primer (common) is unlabeled, and is paired with each of all forward primes for use in PCR. The labeled sites in the primers (Internal), although denoted as T in bold face, may not be limited in terms of site and base. Also, combination of the fluorescent dyes is not limited. The fluorescent dyes labeled at the 5' end and the internal site may be, however, preferably combined so as to demonstrate different fluorescence wavelengths when excited at the same wavelength.

PCR with use of the base sequence of EGFR gene as a target template was conducted with a mixture containing 5 µL of 10× ExTaq buffer (Takara Bio Inc.), 0.25 µL of ExTaq (Takara Bio Inc.), 4 µL of dNTP (10 mM), 1.25 µL of the template DNA (100 fmol/uL), 1.5 µL of the aforementioned primers, and 36.5 µL of D.W., in a thermal cycler operated at 94°C for 5 minutes -> (94°C for 10 seconds -> 55°C for 30 seconds -> 72°C for 1 minute) × 25 cycles -> 72°C for 7 minutes. The sample solution after the reaction was purified with an alkaline phosphatase (Takara Bio Inc.), and then analyzed with use of a capillary electrophoresis (CE) sequencer DS3000 (Hitachi High-Tech Corporation).

After PCR with use of the individual primers, and electrophoresis by CE, relative fluorescence intensity was calculated from the observed fluorescence intensity while assuming a peak value as 100%. Comparative results of the fluorescence spectra are shown in FIG. 3B. From the drawing teaching that the multi-labeling of the primers yielded different fluorescence spectra, it was confirmed that the multi-labeled primers labeled with a combination of currently available fluorescent dyes resulted in increase of the kinds of fluorescent dye different from the currently available fluorescent dyes, thereby confirming the effect.

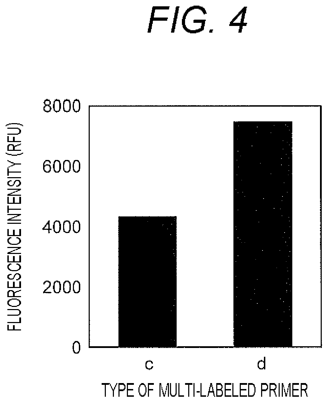

FIG. 4 is a graph showing that multi-labeling of the primers in Example 1 individually with a plurality of fluorescent dyes can enhance the fluorescence intensity. Used herein are the primers listed in FIG. 3A, having the internal site(s) labeled with one molecule of Cy3 (c), and two molecules of Cy3 (d). As a result of PCR, analyses with use of the CE sequencer, and comparison of the fluorescence intensity conducted in the same way as described above, the primer (d) internally labeled with doubled fluorescence was found to demonstrate fluorescence intensity approximately twice as large as that of the primer (c) internally labeled with a single fluorescence, confirming an effect of the multi-labeled primer.

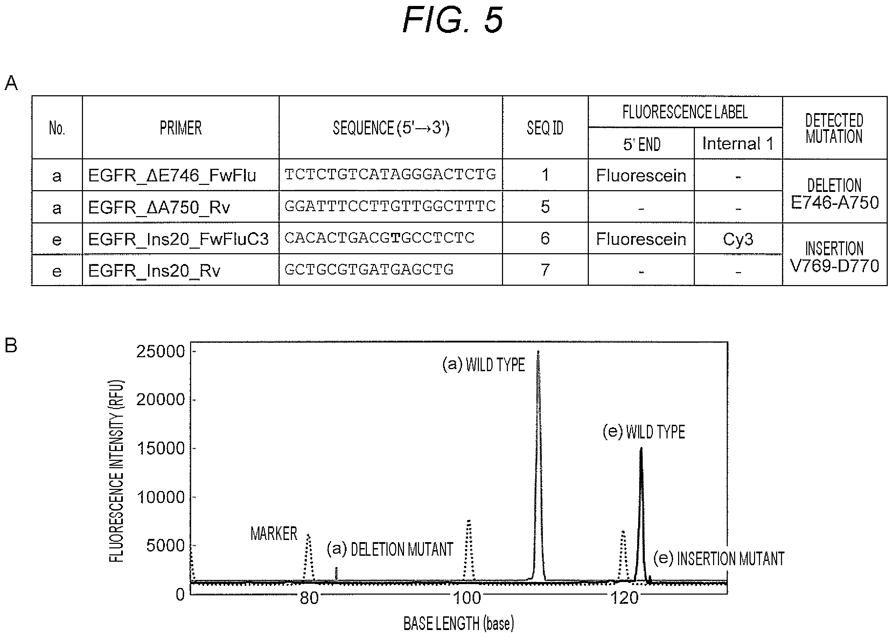

FIG. 5 illustrates an exemplary detection of two different target nucleic acids at a time, with use of multi-labeled primers. Although both target nucleic acids are EGFR genes, one has exon 19 and the other has exon 20 as the target nucleic acids, allowed for detection of different sequences (mutations). Primers prepared herein include a forward primer (a) for detecting a genetic mutation in EGFR resulted from deletion at positions from 746 to 750 in exon 19, labeled with fluorescein at the 5' end; a forward primer (e) for detecting a genetic mutation in EGFR resulted from insertion between positions 769 and 770 in exon 20, labeled with fluorescein at the 5' end, and labeled with Cy3 at the internal site; and unlabeled reverse primers corresponded to each of the forward primers (see FIG. 5A). The internal sites of labeling, although denoted as T in bold face, may not be limited in terms of site and base. Also, combination of the fluorescent dyes may not be limited.

PCR with use of the individual genetic sequences as a target template was conducted with a mixture containing 5 µL of 10× ExTaq buffer (Takara Bio Inc.), 0.25 µL of ExTaq (Takara Bio Inc.), 4 µL of dNTP (10 mM), 1.25 µL of the template DNA mixed liquid (100 fmol/uL of the individual wild types + 10 fmol/uL of the individual mutants), 1.5 µL of mixed liquid of the aforementioned primers, and 36.5 µL of D.W., in a thermal cycler operated at 94°C for 5 minutes -> (94°C for 10 seconds -> 55°C for 30 seconds -> 72°C for 1 minute) × 25 cycles -> 72°C for 7 minutes. The sample solution after the reaction was purified with an alkaline phosphatase (Takara Bio Inc.), and then analyzed with use of a CE sequencer DS3000 (Hitachi High-Tech Corporation). The results showed that, with use of the multi-labeled primers, the wild type and mutant of each of a plurality of types of target nucleic acids were detectable as different fluorescent dyes at a time, confirming an effect of the multi-labeled primer.

### [Example 2]

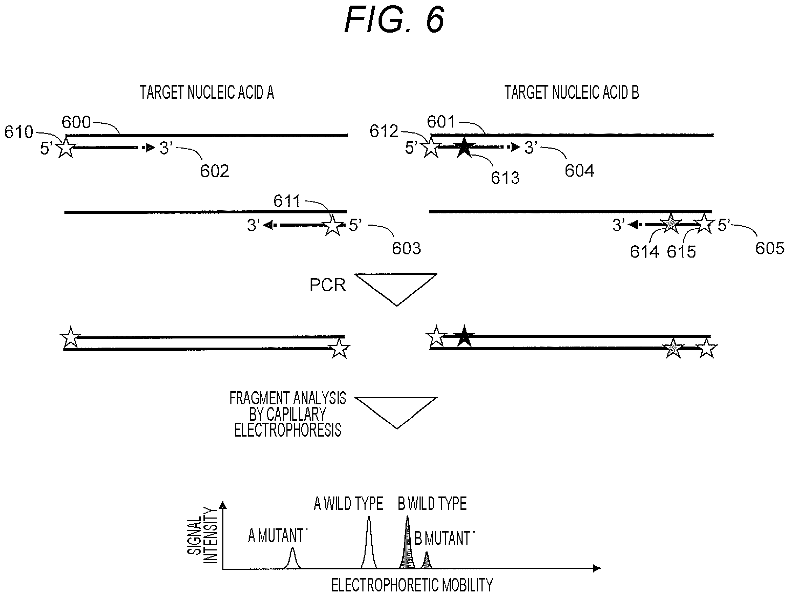

FIG. 6 is a schematic drawing illustrating a fragment analysis method based on capillary electrophoresis, according to Example 2. The drawing illustrates a case where two types of target nucleic acids A and B are contained in the same sample. Note that the number of types of the target nucleic acid is not limited to two.

Used herein are a forward primer 602 and a reverse primer 603 designed complementary to a target nucleic acid A 600 that contains a mutation to be detected; and a forward primer 604 and a reverse primer 605 designed complementary to a target nucleic acid B 601 that contains another mutation to be detected. The forward primer 602 and the reverse primer 603 have fluorescent dyes 610 and 611, respectively, labeled at the 5' ends; meanwhile the forward primer 604 and the reverse primer 605 have fluorescent dyes 612 and 615, respectively, labeled at the 5' ends, and additional fluorescent dyes 613 and 614, respectively, labeled within (Internal) the primers. The amplification by PCR conducted with use of such primers is followed by phoretic separation by capillary electrophoresis (CE). A capillary for the capillary electrophoresis is packed with a polymer for non-denaturating analysis, and Conformational Analysis Polymer from Thermo Fisher Scientific K.K., for example, may be used. Fluorescence detection concurrently with the phoretic separation can finally detect different target nucleic acids and individually correspondent genetic mutations at a time. FIG. 6 illustrates the target nucleic acid A whose mutation is deletion, and the target nucleic acid B whose mutation is insertion. The fluorescent dyes illustrated in the drawing may be of the same kind, or of different kinds. Combination of the fluorescent dyes may not be limited. The fluorescent dyes labeled at the 5' end and the internal site may be, however, preferably combined so as to demonstrate different fluorescence wavelengths when excited at the same wavelength.

### [Example 3]

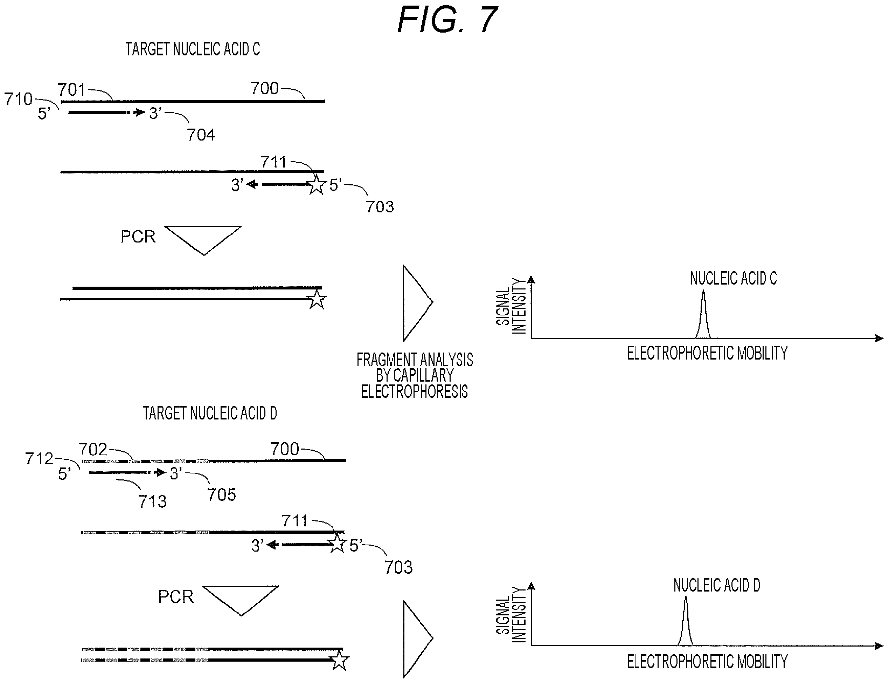

FIG. 7 is a drawing illustrating a conventional fragment analysis method based on capillary electrophoresis. The drawing illustrates detection of fusion mutation.

Both target nucleic acids C and D, containing a fusion mutation to be detected, have a sequence derived from the same gene 700 on the reverse primer sides, meanwhile have the partners of the fusion mutation derived from gene 701 and gene 702 (on forward primer sides), respectively. Since the number of kinds of partner genes for fusion mutation is not always two, so that the conventional method has conducted PCR with use of a fluorescent dye 711 to label the primer (703 in the drawing) designed complementary to the common sequence 700, meanwhile with use of an unlabeled primer (an unlabeled primer 704 designed complementary to the sequence 701, or an unlabeled primer 705 designed complementary to sequence 702) on the side of the gene to be fused (701 and 702 in the drawing), and has then subjected the amplified products to phoretic separation by capillary electrophoresis. As a result, only the mutant having caused fusion mutation may be detected, while leaving the wild-type not having caused fusion unamplified. In this method, the type of gene to be fused (701 or 702 in the drawing) may be determined by the size of the amplified product (the length of the nucleic acid).

The conventional method was, however, difficult to determine whether the detected mutation is an intended one or not, since only one strand derived from the either gene is labeled with the fluorescent dye. Moreover, some sample has limitation in terms of the base length that can be amplified by PCR, so that it would be difficult to detect a plurality of types of fusion mutation, only with reference to the size of the amplification product (the length of the nucleic acid).

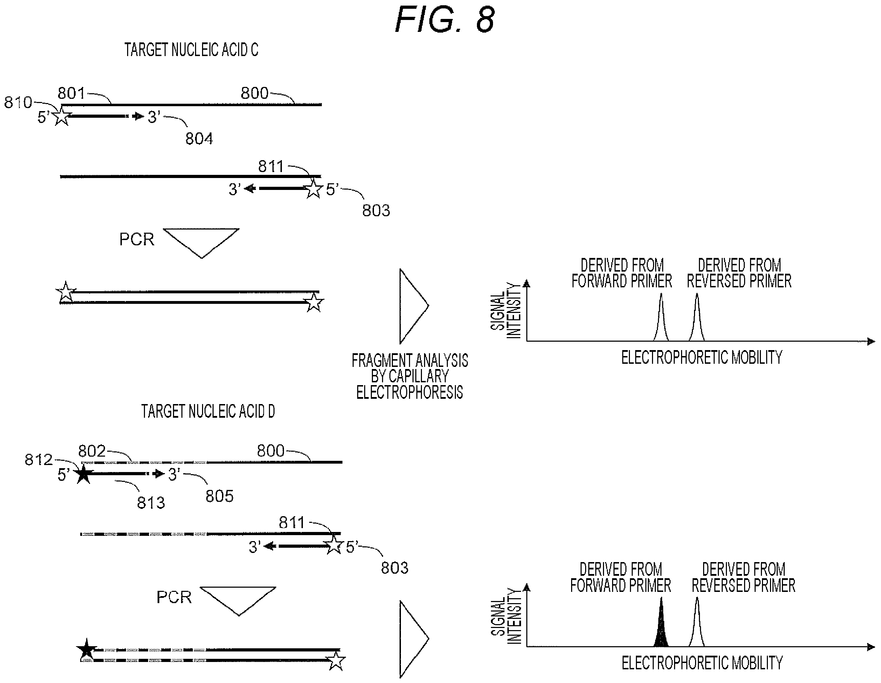

FIG. 8 is a schematic drawing illustrating a fragment analysis method based on capillary electrophoresis, according to Example 3 of the present invention. The drawing schematically illustrates a technique of detecting fusion mutation, with use of multi-labeling in which both the forward primer and the reverse primer are labeled with fluorescence.

Both target nucleic acids C and D, containing a fusion mutation to be detected, have a sequence derived from the same gene 800 on the reverse primer sides, meanwhile have the partners of the fusion mutation derived from gene 801 and gene 802, respectively. Used herein are a reverse primer 803 designed complementary to the common gene 800, a forward primer 804 designed complementary to the sequence 801, and a forward primer 805 designed complementary to the sequence 802. The reverse primer 803 has a fluorescent dye 811 labeled at the 5' end, meanwhile the forward primers 804 and 805 have fluorescent dyes 810 and 812, respectively, labeled at the 5' ends. The fluorescent dyes 810 and 812 used herein are different ones. Either one of them may, however, be the same as the fluorescent dye 811 on 803. The number of molecules of fluorescent dyes to be labeled on each primer is not always one.

After amplified by PCR with use of such primers and dissociating into single-stranded DNAs, the DNAs are phoretically separated by the capillary electrophoresis (CE). A capillary for the capillary electrophoresis is packed with a polymer, and POP4 from Thermo Fisher Scientific K.K., for example, may be used. Fluorescence detection concurrently with the phoretic separation can finally detect the target nucleic acid extended from the forward primer, and the target nucleic acid extended from the reverse primer at a time.

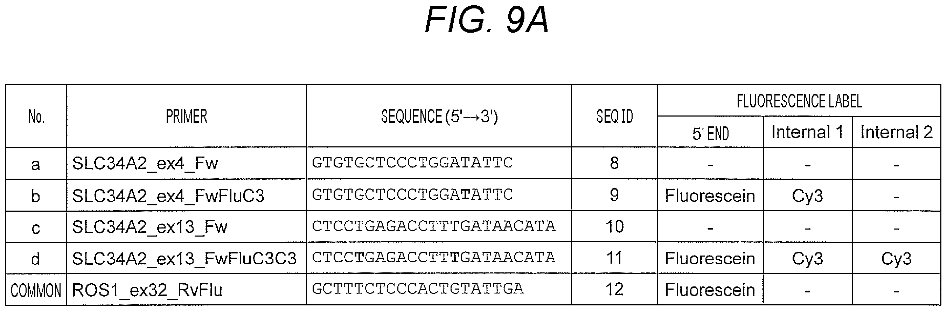

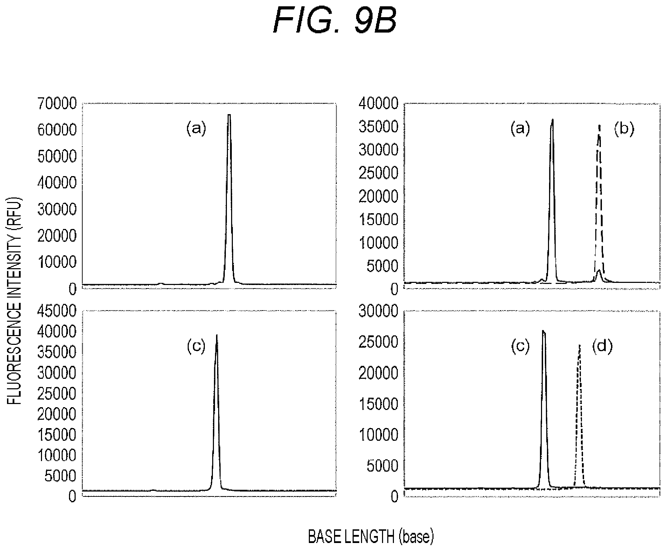

FIGS. 9A and 9B show an effect of detection of fusion mutation, demonstrated by multi-labeling with use of a forward primer and a reverse primer both labeled with fluorescence. Illustrated herein is detection of ROS1-SLC34A2 gene used as the target sequence, which is a fusion mutation gene known as one of driver genes of lung cancer. FIG. 9A lists the primers used herein and the labels therefor. The reverse primer is designed complementary to ROS1, and has fluorescein labeled at the 5'. Each of forward primers a and b has a partner gene for fusion mutation designed complementary to SLC34A2 exon 4, and each of forward primers c and d has a partner gene for fusion mutation designed complementary to SLC34A2 exon 13. The forward primers b and d were prepared by labeling the 5' end with fluorescein, and the internal site(s) with Cy3. The internal sites of labeling, although denoted as T in bold face, may not be limited in terms of site and base. Also, combination of the fluorescent dyes may not be limited.

PCR with use of the base sequence of ROS1-SLC34A2 gene as a target template was conducted with a mixture containing 5 µL of 10× ExTaq buffer (Takara Bio Inc.), 0.25 µL of ExTaq (Takara Bio Inc.), 4 µL of dNTP (10 mM), 1.25 µL of the template DNA (100 finoFuL), 1.5 µL of the aforementioned primers (FIG. 9A), and 36.5 µL of D.W., in a thermal cycler operated at 94°C for 5 minutes -> (94°C for 10 seconds -> 55°C for 30 seconds -> 72°C for 1 minute) × 25 cycles -> 72°C for 7 minutes. The sample solution after the reaction was purified with an alkaline phosphatase (Takara Bio Inc.), and then analyzed with use of a capillary electrophoresis (CE) sequencer DS3000 (Hitachi High-Tech Corporation).

The size of each amplification product is 119 bases for ROS 1-SLC34A2 exon 4, and 118 bases for exon 13. The target gene is detectable as shown in FIG. 9B when only the common primer (reverse primer) is labeled. It may be, however, difficult to identify the fused partner gene from the base length, due to almost the same length ((a) and (c) in FIG. 9B). In contrast, after amplification with use of the forward primer and the reverse primer both labeled and the phoresis, signals of the products amplified with the individual primers can be detected, as shown in FIG. 9B ((a) to (d) in FIG. 9B). The thus split signals are attributable to difference in sequences amplified from the forward primer and from the reverse primer, thus differentiating the mobility. (GeneMapper (registered trademark), Software Version 3.7 User Guide). Use of the forward primer and the reverse primer, both labeled with fluorescence, can yield an effect that not only the target gene is detectable, but also the fused partner gene may be identified more easily.

### [Example 4]

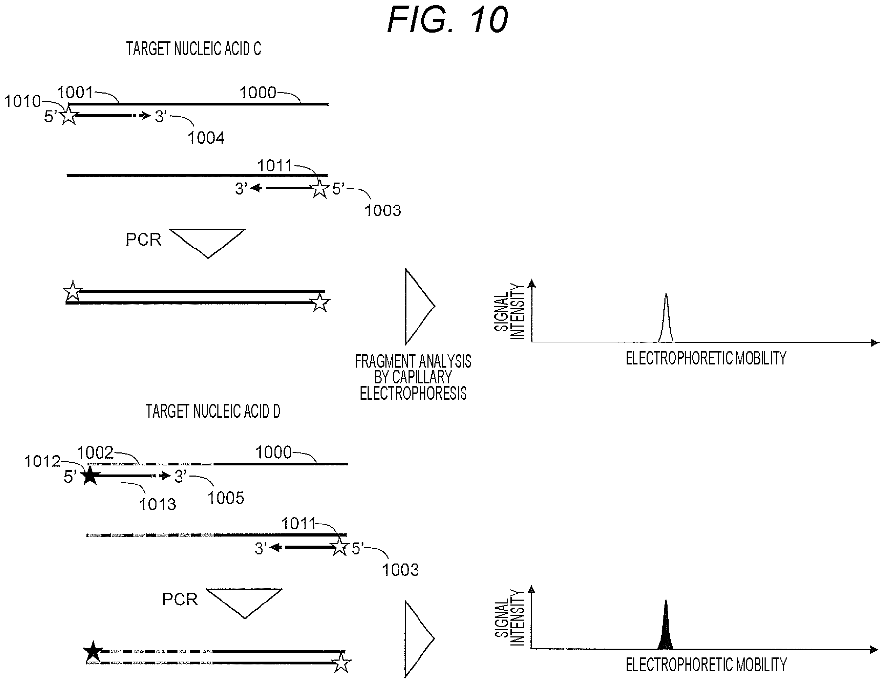

FIG. 10 is a schematic drawing illustrating a fragment analysis method based on capillary electrophoresis, according to Example 4 of the present invention. The drawing schematically illustrates a technique of detecting fusion mutation, with use of multi-labeling in which both the forward primer and the reverse primer are labeled with fluorescence.

Both target nucleic acids C and D, containing a fusion mutation to be detected, have a sequence derived from the same gene 1000 on the reverse primer sides, meanwhile have the partners of the fusion mutation derived from gene 1001 and gene 1002, respectively. Used herein are a reverse primer 1003 designed complementary to the common gene 1000, a forward primer 1004 designed complementary to the sequence 1001, and a forward primer 1005 designed complementary to the sequence 1002. The reverse primer 1003 has a fluorescent dye 1011 labeled at the 5' end, meanwhile the forward primers 1004 and 1005 have fluorescent dyes 1010 and 1012, respectively, labeled at the 5' ends. The fluorescent dyes 1010 and 1012 used herein are different ones. Either one of them may, however, be the same as the fluorescent dye 1011 on 1003. The number of molecules of fluorescent dyes to be labeled on each primer is not always one.

The amplification by PCR conducted with use of such primers is followed by phoretic separation by capillary electrophoresis (CE). A capillary for the capillary electrophoresis is packed with a polymer for non-denaturating analysis, and Conformational Analysis Polymer (CAP) from Thermo Fisher Scientific K.K., for example, may be used. Fluorescence detection concurrently with the phoretic separation can finally detect fusion mutation. Since different fluorescence spectra are obtainable depending on the partners to be fused, the method can determine the fusion mutation, as well as identify the fused partner gene. The fluorescent dyes illustrated in the drawing may be of the same kind, or of different kinds. Combination of the fluorescent dyes may not be limited. The fluorescent dyes labeled at the 5' end and the internal site may be, however, preferably combined so as to demonstrate different fluorescence wavelengths when excited at the same wavelength.

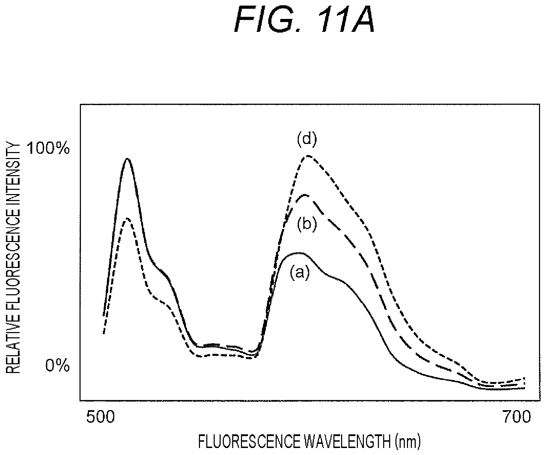

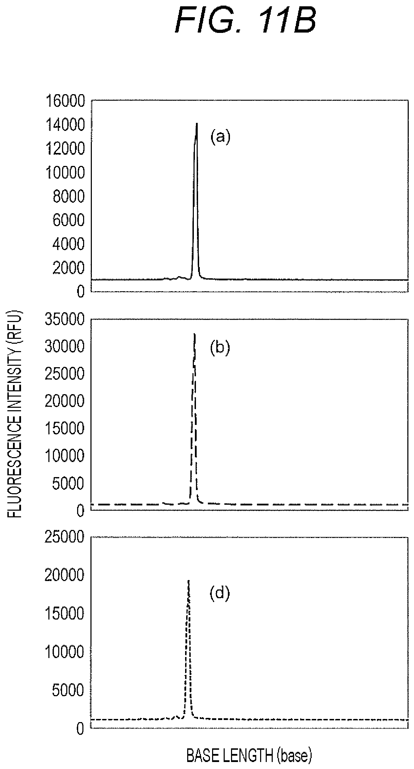

FIGS. 11A and 11B show effects of detection of fusion mutations by multi-labeling with use of a forward primer and a reverse primer both labeled with fluorescence. Illustrated herein is detection of ROS1-SLC34A2 gene used as the target sequence, which is a fusion mutation gene known as one of driver genes of lung cancer. FIG. 9A lists the primers used herein and the labels therefor. The reverse primer is designed complementary to ROS1, and has fluorescein labeled at the 5'. Each of forward primers a and b has a partner gene for fusion mutation designed complementary to SLC34A2 exon 4, and each of forward primers c and d has a partner gene for fusion mutation designed complementary to SLC34A2 exon 13. The forward primers b and d were prepared by labeling the 5' end with fluorescein, and the internal site(s) with Cy3. The internal sites of labeling, although denoted as T in bold face, may not be limited in terms of site and base. Also, combination of the fluorescent dyes may not be limited.

PCR with use of the base sequence of ROS1-SLC34A2 gene as a target template was conducted with a mixture containing 5 µL of 10× ExTaq buffer (Takara Bio Inc.), 0.25 µL of ExTaq (Takara Bio Inc.), 4 µL of dNTP (10 mM), 1.25 µL of the template DNA (100 finoFuL), 1.5 µL of the aforementioned primers (FIG. 9A), and 36.5 µL of D.W., in a thermal cycler operated at 94°C for 5 minutes -> (94°C for 10 seconds -> 55°C for 30 seconds -> 72°C for 1 minute) × 25 cycles -> 72°C for 7 minutes. The sample solution after the reaction was purified with an alkaline phosphatase (Takara Bio Inc.), and then analyzed under non-denaturating conditions with use of a capillary electrophoresis (CE) sequencer DS3000 (Hitachi High-Tech Corporation).

The size of each amplification product is 119 bases for ROS1-SLC34A2 exon 4, and 118 bases for exon 13. The target gene is detectable as shown in FIG. 9B when only the common primer (reverse primer) is labeled. It may be, however, difficult to identify the fused partner gene from the base length, due to almost the same length, and the same fluorescent dye ((a) and (c) in FIG. 9B).

On the other hand, after PCR with use of the forward primer and the reverse primer both labeled, and non-denaturating electrophoresis by CE, relative fluorescence intensity was calculated from the observed fluorescence intensity while assuming a peak value as 100%. Comparative results of the fluorescence spectra are shown in FIG. 11A. From the drawing indicating that the multi-labeling of the primers, in which both the forward primer and the reverse primer are labeled, yielded different fluorescence spectra, it was confirmed that the multi-labeled primers labeled with a combination of currently available fluorescent dyes resulted in increase of the kinds of fluorescent dye different from the currently available fluorescent dyes, thereby confirming the effect.

FIG. 11B shows results of amplification with use of the multi-labeled primers, followed by CE under non-denaturating conditions. The fusion gene may be identifed from a fact that the case (a) where only the reverse primer was fluorescently labeled, and the case (b) where both the forward primer and the reverse primer were fluorescently labeled (b), can be detected as different fluorescent dyes. The fused partner gene may also be identified even for fusion mutation genes having different partners but the same base length, since they are detectable as different fluorescent dyes. The effect of the multi-labeled primer was confirmed from the findings described above.

All publications and the patent specification recited herein constitute, as they are, a part of the description of this patent specification.

### Reference Signs List

100, 200, 203, 600, 601: target nucleic acid
700, 701, 702, 800, 801, 802, 1000, 1001, 1002: gene (fusion mutation)
101, 201, 204, 205, 610, 611, 612, 613, 614, 615, 711, 810, 811, 812, 1010, 1011, 1012: labeled fluorescent dye
102, 103, 202, 206, 207, 208, 602, 603, 604, 605, 703, 704, 705, 803, 804, 805, 1003, 1004, 1005: primer

## Claims

1. A method for detecting a mutation in a test nucleic acid, the method comprising:
providing a first primer set, comprising at least one labeled first primer, for amplifying a nucleic acid with a first mutation;
providing a second primer set, comprising at least one labeled second primer, for amplifying a nucleic acid with a second mutation;
amplifying the test nucleic acid with use of the first primer set and the second primer set; and
subjecting an obtained amplification product to capillary electrophoresis to analyze the mutation in the test nucleic acid,
the first primer and the second primer being individually labeled with combinations of different numbers and/or kinds of fluorescent dyes.

2. The method according to claim 1, wherein the first primer set further comprises a labeled first' primer; and/or the second primer set further comprises a labeled second' primer.

3. The method according to claim 2, wherein the first primer and the first' primer are individually labeled with a combination of the same number and/or kind of fluorescent dyes, or with combinations of different numbers and/or kinds of fluorescent dyes; and/or the second primer and the second' primer are individually labeled with a combination of the same number and/or kind of fluorescent dyes, or with combinations of different numbers and/or kinds of fluorescent dyes.

4. The method according to claim 1, further comprising providing an n-th primer set, comprising at least one labeled n-th primer, for amplifying a nucleic acid with a mutation different from the first mutation and the second mutation, where n represents an integer of 3 to 100, and the n-th primer is labeled with a combination of fluorescent dyes whose number and/or kind are different from those in the first primer and the second primer.

5. The method according to claim 1, wherein the 5' end of the first primer and/or the second primer is labeled with a fluorescent dye; and/or a base within a sequence of the first primer and/or the second primer is labeled with a fluorescent dye.

6. The method according to claim 1, wherein the mutation comprises a structural polymorphism mutation.

7. The method according to claim 1, wherein, after amplifying the test nucleic acid, the obtained product is dissociated into a single-stranded nucleic acid, and then subjected to the capillary electrophoresis.

8. A kit used for detecting a mutation in a nucleic acid by capillary electrophoresis, the kit comprising:
a first primer set, comprising at least one labeled first primer, for amplifying a nucleic acid with a first mutation; and
a second primer set, comprising at least one labeled second primer, for amplifying a nucleic acid with a second mutation,
the first primer and the second primer being individually labeled with combinations of different numbers and/or kinds of fluorescent dyes.

9. The kit according to claim 8, wherein the first primer set further comprises a labeled first' primer; and/or the second primer set further comprises a labeled second' primer.

10. The kit according to claim 9, wherein the first primer and the first' primer are individually labeled with a combination of the same number and/or kind of fluorescent dyes, or with combinations of different numbers and/or kinds of fluorescent dyes; and/or the second primer and the second' primer are individually labeled with a combination of the same number and/or kind of fluorescent dyes, or with combinations of different numbers and/or kinds of fluorescent dyes.

11. The kit according to claim 8, further comprising an n-th primer set, comprising at least one labeled n-th primer, for amplifying a nucleic acid with a mutation different from the first mutation and the second mutation, where n represents an integer of 3 to 100, and the n-th primer is labeled with a combination of fluorescent dyes whose number and/or kind are different from those in the first primer and the second primer.

12. The kit according to claim 8, wherein the 5' end of the first primer and/or the second primer is labeled with a fluorescent dye; and/or a base within the first primer and/or the second primer is labeled with a fluorescent dye.

13. The kit according to claim 8, wherein the mutation comprises a structural polymorphism mutation.
